# FASCICULE DE BREVET EUROPEEN

(11) **EP 1 954 839 B1**
(45) Date de publication et mention de la délivrance du brevet: **20.10.2010**
(21) Numéro de dépôt: 06842029.8
(22) Date de dépôt: 23.11.2006
(51) Int. Cl.: C12Q 1/70

(54) **OLIGONUCLEOTIDES, UTILISATION, METHODE DE DETECTION ET KIT PERMETTANT DE DIAGNOSTIQUER LA PRESENCE DES GENES H5 ET N1 DU VIRUS D'INFLUENZA A**
OLIGONUKLEOTIDE, VERWENDUNG DAVON, ERKENNUNGSVERFAHREN UND KIT ZUR FESTSTELLUNG DER ANWESENHEIT VON H5- UND N1-GENEN DES INFLUENZA-VIRUS
OLIGONUCLEOTIDES, USE THEREOF, DETECTING METHOD AND KIT FOR DIAGNOSING THE PRESENCE OF H5 AND N1 GENES OF THE INFLUENZA A VIRUS

(30) Priorité: 25.11.2005 FR 0511974; 30.01.2006 FR 0600843
(43) Date de publication de la demande: 13.08.2008
(73) Titulaire: BIOMERIEUX D.P.I., 69280 MARCY L'ETOILE (FR)
(72) Inventeur: LEFEUVRE, Aurélie, F-38000 Grenoble (FR); TELLES, Jean-Noël, F-69530 Brignais (FR); VERNET, Guy, F-69540 Irigny (FR)
(86) Numéro de dépôt international: PCT/FR2006/051218
(87) Numéro de publication internationale: WO 2007/060366

(56) Documents cités:
- WO-A-02/29118
- WO-A-2005/121367
- PAYUNGPORN SUNCHAI ET AL: "Single-step multiplex reverse transcription-polymerase chain reaction (RT-PCR) for influenza A virus subtype H5N1 detection" VIRAL IMMUNOLOGY, vol. 17, no. 4, janvier 2004 (2004-01), pages 588-593, XP002399974 ISSN: 0882-8245 cité dans la demande
- GREIJER A E ET AL: "Multiplex real-time NASBA for monitoring expression dynamics of human cytomegalovirus encoded IE1 and pp67 RNA" JOURNAL OF CLINICAL VIROLOGY 2002 NETHERLANDS, vol. 24, no. 1-2, 2002, pages 57-66, XP002399982 ISSN: 1386-6532
- COLLINS RICHARD A ET AL: "Detection of highly pathogenic and low pathogenic avian influenza subtype H5 (Eurasian lineage) using NASBA" JOURNAL OF VIROLOGICAL METHODS, vol. 103, no. 2, mai 2002 (2002-05), pages 213-225, XP002399975 ISSN: 0166-0934 cité dans la demande
- DATABASE WPI Week 200613 Derwent Publications Ltd., London, GB; AN 2006-119038 XP002400009 & CN 1 635 163 A (UNIV CHINA AGRIC) 6 juillet 2005 (2005-07-06)

### SEQUENCE LISTING

<110> bioMérieux
<120> Oligonucléotides, utilisation, méthode de détection et kit permettant de diagnostiquer la présence des gènes H5 et N1 du virus d'Influenza A
<130> H5N1 PI
<140> FR06/00843
   <141> 2006-01-30
<150> FR05/11974
   <151> 2005-11-25
<160> 10
<170> PatentIn version 3.3
<210> 1
   <211> 19
   <212> DNA
   <213> Influenza A virus
<400> 1
   tgtatgttgt ggaatggca 19
<210> 2
   <211> 18
   <212> DNA
   <213> Influenza A virus
<400> 2
   gccgaatgat gccatcaa 18
<210> 3
   <211> 18
   <212> DNA
   <213> Influenza A virus
<400> 3
   cgtggattgt ctccgaaa 18
<210> 4
   <211> 21
   <212> DNA
   <213> Influenza A virus
<400> 4
   ggaatgctcc tgttatcctg a 21
<210> 5
   <211> 45
   <212> DNA
   <213> Influenza A virus
<400> 5
   aattctaata cgactcacta taggggtgta tgttgtggaa tggca 45
<210> 6
   <211> 44
   <212> DNA
   <213> Influenza A virus
<400> 6
   aattctaata cgactcacta taggggcgtg gattgtctcc gaaa 44
<210> 7
   <211> 22
   <212> DNA
   <213> Influenza A virus
<400> 7
   acaccaagtg tcaaactcca at 22
<210> 8
   <211> 25
   <212> DNA
   <213> Influenza A virus
<400> 8
   gcgaaatcac atgtgtgtgc aggga 25
<210> 9
   <211> 34
   <212> DNA
   <213> Influenza A virus
<400> 9
   cgatcgacac caagtgtcaa actccaatcg atcg 34
<210> 10
   <211> 37
   <212> DNA
   <213> Influenza A virus
<400> 10
   cgatcggcga aatcacatgt gtgtgcaggg acgatcg 37

## Description

La présente invention concerne un procédé de marquage d'acides nucléiques en présence d'au moins un support solide.

La présente invention concerne des oligonucléotides destinés à permettre l'amplification et la détection de deux séquences cibles localisées respectivement dans les gènes H5 et N1 du génome du virus *Influenza* A.

L'invention concerne également l'utilisation de ces oligonucléotides, une méthode de détection et un kit permettant de diagnostiquer la présence des gènes H5 et N1 du virus *Influenza* A.

Parmi les techniques classiques utilisées en routine pour le diagnostic de la grippe, on peut citer l'agglutination, l'inhibition d'agglutination ou la diffusion en gel d'agar. Ces méthodes sont communément employées et permettent la caractérisation du virus A de la grippe.

Une alternative possible à ces méthodes est la culture virale dans des oeufs au stade d'embryon ou dans des cellules MDCK, selon le protocole du manuel de l'OIE (Office International des Epizooties). Cependant, plusieurs jours sont nécessaires pour obtenir les résultats de caractérisation, délai parfois incompatible avec les besoins ou urgences cliniques.

Des techniques d'ELISA pour la détection d'anticorps ou d'antigène ou des tests d'immunofluorescence ont été également massivement développées mais ces méthodes de détection, dites immunologiques, sont souvent moins sensibles et moins spécifiques que la culture virale classique.

L'émergence récente d'une forme hautement pathogénique du virus de la grippe aviaire, le sous-type H5N1, a donc fortement relancé le besoin d'un test de diagnostic rapide, spécifique et très sensible. Pour cette raison, les différentes méthodes précédemment énumérées ont été progressivement remplacées par des techniques dites moléculaires, telle que la technique de RT-PCR (transcription inverse associée à une réaction de polymérisation en chaîne). La RT-PCR, plus sensible, ne nécessite pas la présence de virus « viable » dans les échantillons, et a ainsi permis le typage et sous-typage des différentes formes du virus de la grippe. Le développement de cette technique en temps réel (« real time RT-PCR ») a également grandement favorisé son utilisation pour le diagnostic du virus de type A.

Par exemple, Whiley D. M. et al. décrivent dans une récente publication (Diagnostic Microbiology and Infectious Diseases (2005), sous presse)*,* un test pour la détection d'un large spectre de sous-types de la grippe dans des échantillons cliniques, basé sur une réaction de RT-PCR faisant intervenir une 5' nucléase. Deux oligonucléotides et une sonde ont été choisis de façon à être homologues au gène codant la protéine M (matrice) de 23 sous-types du virus A. Ce test permet donc la détection du virus *Influenza* A dans des échantillons cliniques mais ne permet pas par contre le sous-typage précis de la forme impliquée.

Ng E.K.O. et al. ont récemment divulgué (Emerging Infectious Diseases (2005) vol. 11 (8), p. 1303-1305) un test basé sur une réaction de RT-PCR en multiplex comprenant deux étapes, utilisant deux jeux d'oligonucléotides et de sondes marquées afin de cibler spécifiquement deux régions du gène HA de H5N1. Ceci a ainsi permis de développer un test rapide et sensible pour détecter directement le sous-type H5 dans des échantillons humains. Ce test a été validé sur des échantillons cliniques provenant de patients infectés à Hong Kong et au Vietnam par le sous-type H5N1 du virus, mais ne permet pas de diagnostiquer directement quel sous-type de virus est impliqué dans l'infection.

Payungporn S. et al. décrivent (Viral Immunology (2004) vol. 17 (4), p. 588-593 et Journal of Virological Methods (2005), sous presse*)* une méthode de détection simultanée des séquences M, H5 et N1 du sous-type H5N1, basée sur un test multiplex RT-PCR en temps réel et en une seule étape. Des oligonucléotides correspondant aux séquences M, H5 et N1, ainsi que différentes sondes TaqMan marquées ont été sélectionnées et utilisées dans le test afin de détecter simultanément trois signaux fluorescents. Les oligonucléotides H5 et N1 ont été choisis à partir de régions invariantes couvrant plus de 50 séquences connues spécifiques du virus H5N1. Cependant, il est à noter que cette méthode de RT-PCR, non isotherme, est parfois assujettie à des contaminations.

Une autre méthode moléculaire pouvant être employée pour détecter la présence d'agent infectieux est la technique NASBA (Nucleic Acid Sequence-Based Amplification). Par exemple, Collins R.A et al. décrivent (Journal of Virological Methods (2002) vol. 103, p.213-225 et Avian Diseases (2003) vol. 47 (3), p. 1069-1074) la détection du sous-type H5 de la grippe aviaire (détection du sous-type hautement pathogénique et faiblement pathogénique) en utilisant la technique NASBA couplée à un système de détection ECL (electrochemiluminescence). La technique NASBA est une méthode d'amplification isotherme d'acides nucléiques faisant intervenir plusieurs activités enzymatiques, qui permet une détection rapide du virus H5. L'amplification d'acides nucléiques par cette voie convient bien aux génomes à ARN, comme le génome du virus de la grippe, grâce à l'introduction de l'étape de transcription inverse directement dans la réaction d'amplification. Néanmoins, même si la méthode décrite dans cette publication permet de détecter et de distinguer les souches faiblement pathogéniques des souches hautement pathogéniques, elle ne permet d'identifier spécifiquement la forme H5N1 du virus. Le document WO 2005/121367 (publié le 22.12.2005 ayant une date de dépôt du 10.06.2005) décrit la séquence d'acide nucléique cattggagtttgacacttggtgtt comme amorce d'amplification pour la détection du virus H5N1.

Un test d'identification du virus *Influenza* A sous sa forme H5N1 utilisant une technique d'amplification transcriptionnelle, particulièrement adaptée à l'amplification et à la détection de virus à ARN, est donc toujours attendu. De plus un test multiplexe, c'est-à-dire permettant l'amplification et la détection simultanément des deux gènes H5 et N1, la technique d'amplification étant une technique d'amplification transcriptionnelle telle que la NASBA, est particulièrement avantageuse.

La présente invention concerne donc une double paire d'oligonucléotides destinée à permettre l'amplification de deux séquences cibles localisées respectivement dans le gène H5 et dans le gène N1 du génome du virus *Influenza* A, la paire d'oligonucléotides destinée à l'amplification du gène H5 consistant en :
- un premier oligonucléotide d'une longueur comprise entre 10 et 50 nucléotides et comprenant au moins un fragment de 10 nucléotides consécutifs issus de :
   SEQ ID No. 1 : TGTATGTTGTGGAATGGCA, ou sa séquence complémentaire, et
- un second oligonucléotide d'une longueur comprise entre 10 et 50 nucléotides et comprenant au moins un fragment de 10 nucléotides consécutifs issus de :
   SEQ ID No. 2 : GCCGAATGATGCCATCAA, ou sa séquence complémentaire, alors que la paire d'oligonucléotides destinée à l'amplification du gène N1 consiste en :
- un premier oligonucléotide d'une longueur comprise entre 10 et 50 nucléotides et comprenant au moins un fragment de 10 nucléotides consécutifs issus de :
   SEQ ID No. 3 : CGTGGATTGTCTCCGAAA, ou sa séquence complémentaire, et
- un second oligonucléotide d'une longueur comprise entre 10 et 50 nucléotides et comprenant au moins un fragment de 10 nucléotides consécutifs issus de :
   SEQ ID No. 4 : GGAATGCTCCTGTTATCCTGA, ou sa séquence complémentaire.

L'invention peut également avoir trait à une paire d'oligonucléotides destinée à permettre l'amplification d'une séquence cible localisée dans le gène H5 du génome du virus *Influenza* A, la paire d'oligonucléotides consistant en :
- un premier oligonucléotide d'une longueur comprise entre 10 et 50 nucléotides et comprenant au moins un fragment de 10 nucléotides consécutifs issus de :
   SEQ ID No. 1 : TGTATGTTGTGGAATGGCA, ou sa séquence complémentaire, et
- un second oligonucléotide d'une longueur comprise entre 10 et 50 nucléotides et comprenant au moins un fragment de 10 nucléotides consécutifs issus de :
   SEQ ID No. 2 : GCCGAATGATGCCATCAA, ou sa séquence complémentaire.

De la même manière, l'invention peut aussi couvrir une paire d'oligonucléotides destinée à permettre l'amplification d'une séquence cible localisée dans le gène N1 du génome du virus *Influenza* A, la paire d'oligonucléotides consistant en :
- un premier oligonucléotide d'une longueur comprise entre 10 et 50 nucléotides et comprenant au moins un fragment de 10 nucléotides consécutifs issus de :
   SEQ ID No. 3: CGTGGATTGTCTCCGAAA, ou sa séquence complémentaire, et
- un second oligonucléotide d'une longueur comprise entre 10 et 50 nucléotides et comprenant au moins un fragment de 10 nucléotides consécutifs issus de :
   SEQ ID No. 4 : GGAATGCTCCTGTTATCCTGA, ou sa séquence complémentaire.

Dans les trois cas de figure précédents, dans la (ou les) paire(s) d'oligonucléotides, le premier oligonucléotide comprend additionnellement une séquence promotrice qui peut être reconnue par une enzyme ARN polymérase ADN dépendante.

Plus précisément, la séquence promotrice qui peut être reconnue par une enzyme ARN polymérase ADN dépendante est une polymérase T7.

En relation avec les deux cas précédents, lorsque cet oligonucléotide, auquel est additionnée la séquence promotrice, permet l'amplification de la séquence cible localisée dans le gène H5, il consiste essentiellement en la séquence suivante :
SEQ ID No. 5 : aattctaatacgactcactataggggTGTATGTTGTGGAATGGCA, ou sa séquence complémentaire. La partie de la séquence en lettres minuscules correspond à la séquence promotrice T7.

De la même manière que précédemment, lorsque cet oligonucléotide permet l'amplification de la séquence cible localisée dans le gène N1, il consiste essentiellement en la séquence suivante :
SEQ ID No. 6 : aattctaatacgactcactataggggCGTGGATTGTCTCCGAAA, ou sa séquence complémentaire.

Dans tous les cas de figure, chaque oligonucléotide peut être d'une longueur comprise entre 12 et 30 nucléotides et comprenant au moins un fragment de 16 nucléotides consécutifs, et préférentiellement d'une longueur comprise entre 15 et 26 nucléotides et comprenant au moins un fragment de 18 nucléotides consécutifs.

L'invention concerne également une paire d'oligonucléotides destinée à être utilisée comme sonde de détection de deux séquences cibles localisées respectivement dans les gènes H5 et N1 du génome du virus *Influenza* A, la sonde destinée à la détection du gène H5 consistant en :
SEQ ID No. 7 : ACACCAAGTGTCAAACTCCAAT, ou sa séquence complémentaire,
alors que la sonde destinée à la détection du gène N1 consiste en :
SEQ ID No. 8 : GCGAAATCACATGTGTGTGCAGGGA, ou sa séquence complémentaire,
chaque séquence comportant au moins un moyen de marquage.

L'invention peut également avoir trait à un oligonucléotide, destiné à être utilisé comme sonde de détection d'une séquence d'acides nucléiques amplifiée résultant de l'amplification d'une séquence cible localisée dans le gène H5 du génome du virus *Influenza* A, ladite amplification étant réalisée par l'intermédiaire d'une paire d'oligonucléotides telle que décrite précédemment, la sonde de détection étant d'une longueur comprise entre 10 et 50 nucléotides et comprenant au moins un fragment de 10 nucléotides consécutifs issus de :
SEQ ID No. 7 : ACACCAAGTGTCAAACTCCAAT, ou sa séquence complémentaire, la séquence comportant au moins un moyen de marquage.

Lorsque l'on souhaite détecter le gène N1 du génome du virus *Influenza* A, l'invention préconise un oligonucléotide, destiné à être utilisé comme sonde de détection d'une séquence d'acides nucléiques amplifiée résultant de l'amplification d'une séquence cible localisée dans ce gène N1, ladite amplification étant réalisée par l'intermédiaire d'une paire d'oligonucléotides telle que décrite précédemment, la sonde de détection étant d'une longueur comprise entre 10 et 50 nucléotides et comprenant au moins un fragment de 10 nucléotides consécutifs issus de :
SEQ ID No. 8 : GCGAAATCACATGTGTGTGCAGGGA, ou sa séquence complémentaire, la séquence comportant au moins un moyen de marquage.

Dans un mode de réalisation intéressant de l'invention, la sonde de détection est constituée par un « molecular beacon », appellé par la suite sonde moléculaire. Les sondes moléculaires sont des sondes de détection sous forme d'oligonucléotides simple brin, qui ont une structure en tige et boucle (stem loop structure) bien connue de l'homme du métier. La boucle contient une séquence sonde complémentaire de la séquence cible (amplicon en général), et la tige est formée par l'hybridation de deux séquences formant bras, qui sont localisées chacune à chaque extrémité de la sonde. Un fluorophore est lié de façon covalente à l'extrémité d'un des deux bras et un absorbeur de fluorescence (quencher) est lié de façon covalente à l'extrémité de l'autre bras. Les sondes moléculaires ne fluorescent pas lorsqu'ils sont libres en solution. Cependant, en présence d'amplicons complémentaires, quand ils s'hybrident à ces cibles, ils subissent un changement conformationnel qui leur permet de fluorescer. En l'absence de cibles, la tige conserve le fluorophore en étroite proximité avec l'absorbeur de fluorescence, ce qui entraîne le transfert de la fluorescence du fluorophore vers l'absorbeur. Ledit absorbeur de fluorescence est un chromophore non fluorescent qui dissipe l'énergie reçue du fluorophore en chaleur. Quand la sonde rencontre une cible moléculaire, il y a formation d'un hybride sonde-cible qui est plus long et plus stable que l'hybride créé par les deux bras de la tige. La rigidité et la longueur de l'hybride sonde-hybride empêchent l'existence simultanément de l'hybride tige. En conséquence, la sonde moléculaire subit une réorganisation conformationnelle spontanée qui force l'hybride tige à se dissocier et le fluorophore et l'absorbeur à s'éloigner l'un de l'autre, ce qui restaure la fluorescence.

Plus précisément la sonde de détection est constituée par une sonde moléculaire préférentiellement constituée par :
SEQ ID No. 9 : [6-FAM]-cgatcgACACCAAGTGTCAAACTCCAATcgatcg-[DabSyl], pour la détection du gène H5,
SEQ ID No. 10 : [6-FAM]-cgatcgGCGAAATCACATGTGTGTGCAGGGAcgatcg-[DabSyl], pour la détection du gène N1.

Lorsque le test que l'on souhaite effectuer et un test multiplexe où la détection des gènes H5 et N1 est simultanée et effectuée dans un unique récipient, il convient d'utiliser deux marqueurs différents. Parmi les marqueurs fluorescents possibles, citons de manière non limitative :
- la fluorescéine (FAM),
- la tétrachloro-6-carboxyfluorescéine (TET),
- la tétraméthylrhodamine (TMR),
- la 5-carboxyrhodamine 6G (RHD),
- la carboxy-rhodamine (ROX), et
- la cyanine 5 (CY5).

Chacune des deux séquences SEQ ID Nos. 9 et 10 ci-dessus sera donc munie d'un de ces marqueurs, les deux marqueurs utilisés étant différents l'un de l'autre afin de permettre une différentiation des signaux détectés.

L'invention propose aussi l'utilisation d'une ou deux paires d'oligonucléotides, telle(s) que décrite(s) précédemment, dans une réaction d'amplification d'acides nucléiques ou comme sonde de détection du génome du virus *Influenza* A suspecté d'être présent dans un échantillon biologique.

L'invention concerne encore une méthode de détection d'acides nucléiques du virus d'*Influenza* A susceptible d'être présent dans un échantillon, dans laquelle l'échantillon est soumis à une réaction d'amplification d'acides nucléiques utilisant une paire d'oligonucléotides, telle que décrite précédemment, en présence des réactifs d'amplification nécessaires à une telle amplification et la présence d'amplicons d'intérêt est détecté.

Cette méthode de détection peut être basée sur une réaction d'amplification RT-PCR.

Alternativement, cette méthode de détection peut être basée sur une technique d'amplification transcriptionnelle. Préférentiellement cette technique est la technique NASBA.

L'invention concerne aussi une méthode d'amplification de deux gènes H5 et N1 du virus d'*Influenza* A susceptible d'être présent dans un échantillon, comprenant les étapes suivantes :
- incuber l'échantillon dans un tampon d'amplification en présence :
   - de deux amorces d'amplification, chacune ayant une longueur comprise entre 10 et 50 nucléotides, l'une comprenant additionnellement une séquence promotrice, l'autre de polarité opposée à l'amorce associée à la séquence promotrice, pour s'hybrider respectivement en amont et en aval d'une zone d'intérêt localisée dans le gène H5 du virus d'*Influenza* A,
   - de deux amorces d'amplification, chacune ayant une longueur comprise entre 10 et 50 nucléotides, l'une comprenant additionnellement une séquence promotrice, l'autre de polarité opposée à l'amorce associée à la séquence promotrice, pour s'hybrider respectivement en amont et en aval d'une zone d'intérêt localisée dans le gène N1 du virus d'*Influenza* A,
- ajouter les réactifs suivants dans l'échantillon:
   - une enzyme ayant une activité RNA dépendant DNA polymérase,
   - une enzyme ayant une activité DNA dépendant DNA polymérase,
   - une enzyme ayant une activité RNase H,
   - une enzyme ayant une activité DNA dépendent RNA polymérase, et
- maintenir le mélange réactionnel ainsi créé sous des conditions appropriées et pendant une durée de temps suffisante pour qu'une amplification ait lieu.

Les enzymes ci-dessus énumérés sont au nombre de quatre mais il est tout à fait possible de recourir à une enzyme ayant deux voir trois des activités ci-dessus mentionnées, dans ce cas l'utilisation de trois voir de deux enzymes reste possible et couverte par l'invention. De plus, d'autres éléments nécessaires à l'établissement d'une amplification sont nécessaires tels que des nucléotides. De tels éléments sont bien connus de l'homme du métier.

Enfin l'invention propose un kit de détection des gènes H5 et N1 du virus *Influenza* A susceptible d'être présent dans un échantillon, contenant :
- deux paires d'oligonucléotides ou amorces d'amplification telles que décrites précédemment pour réaliser l'amplification des deux régions d'intérêt H5 et N1,
- deux oligonucléotides marqués ou pouvant être marqués, tels que décrits précédemment, et ayant une séquence d'acide nucléique substantiellement complémentaire avec au moins une partie de la séquence d'acide nucléique amplifiée H5 ou N1,
- des réactifs nécessaires à la réalisation d'une réaction d'amplification.

Par « substantiellement complémentaire » on entend qu'une hybridation est réalisée entre un oligonucléotide marqué ou pouvant être marqué, autrement appelé sonde de détection, et au moins une partie de la séquence d'acide nucléique amplifiée ou amplicon, cette hybridation étant spécifique et sélective de manière suffisante pour permettre la détection de l'amplicon d'intérêt.

D'autre part, les réactifs nécessaires à la réalisation d'une réaction d'amplification sont des réactifs permettant une amplification NASBA.

Par « marqueur détectable », on entend au moins un marqueur capable de générer directement un signal détectable. Par exemple la présence de biotine est considéré comme un marquage direct, car elle est détectable, même s'il est possible de l'associer ultérieurement avec de la streptavidine marquée. Une liste non limitative de ces marqueurs suit :
- les enzymes qui produisent un signal détectable par exemple par colorimétrie, fluorescence, luminescence, comme la péroxydase de raifort, la phosphatase alcaline, la β-galactosidase, la glucose-6-phosphate déshydrogénase,
- les chromophores comme les composés fluorescents, luminescents, colorants,
- les groupements à densité électronique détectable par microscopie électronique ou par leur propriété électrique comme la conductivité, l'ampérométrie, la voltamétrie, l'impédance,
- les groupements détectables, par exemple dont les molécules sont de tailles suffisantes pour induire des modifications détectables de leurs caractéristiques physiques et/ou chimiques, cette détection peut être réalisée par des méthodes optiques comme la diffraction, la résonance plasmon de surface, la variation de surface, la variation d'angle de contact ou des méthodes physiques comme la spectroscopie de force atomique, l'effet tunnel,
- les molécules radioactives comme le ³²P, le ³⁵S ou le ¹²⁵I.
De préférence, le marqueur n'est pas un marqueur radioactif pour éviter les problèmes de sécurité liés à ces marqueurs.

Dans un mode de réalisation particulier de la présente invention le marqueur est détectable électrochimiquement, et en particulier le marqueur est un dérivé d'un complexe de fer, comme un ferrocène.

Le terme « acide nucléique » signifie un enchaînement d'au moins deux désoxyribonucléotides ou ribonucléotides comprenant éventuellement au moins un nucléotide modifié, par exemple au moins un nucléotide comportant une base modifiée, telle que l'inosine, la méthyl-5-désoxycytidine, la diméthylamino-5-désoxyuridine, la désoxyuridine, la diamino-2,6-purine, la bromo-5-désoxyuridine ou toute autre base modifiée permettant l'hybridation. Ce polynucléotide peut aussi être modifié au niveau de la liaison internucléotidique comme par exemple les phosphorothioates, les H-phosphonates, les alkyl-phosphonates, au niveau du squelette comme par exemple les alpha-oligonucléotides ( FR 2 607 507) ou les PNA (M. Egholm et al., J. Am. Chem. Soc., 114, 1895-1897, 1992 ou les 2' O-alkyl ribose et les LNA (BW, Sun et al., Biochemistry, 4160-4169, 43, 2004). L'acide nucléique peut être naturel ou synthétique, un oligonucléotide, un polynucléotide, un fragment d'acide nucléique, un ARN ribosomique, un ARN messager, un ARN de transfert, un acide nucléique obtenu par une technique d'amplification enzymatique telle que :
- PCR (Polymerase Chain Reaction), décrite dans les brevets US-A-4,683,195, US-A-4,683,202 et US-A-4,800,159, et sa dérivée RT-PCR (Reverse Transcription PCR), notamment dans un format en une étape, tel que décrit dans le brevet EP-B-0.569.272,
- LCR (Ligase Chain Reaction), exposée par exemple dans la demande de brevet EP-A-0.201.184,
- RCR (Repair Chain Reaction), décrite dans la demande de brevet WO-A-90/01069,
- 3SR (Self Sustained Sequence Replication) avec la demande de brevet WO-A-90/06995,
- NASBA (Nucleic Acid Sequence-Based Amplification) avec la demande de brevet WO-A-91/02818, et
- TMA (Transcription Mediated Amplification) avec le brevet US-A-5,399,491.
- RCA (Rolling Circle Amplification (US-6,576,448).
On parle alors d'amplicons pour désigner les acides nucléiques générés par une technique d'amplification enzymatique.

Chacune de ces modifications peut être prise en combinaison.

Les étapes d'amplification et de détection ci-dessus exposées peuvent être précédées par une étape de purification. Par « étape de purification », on entend notamment la séparation entre les acides nucléiques des micro-organismes et les constituants cellulaires relargués dans l'étape de lyse qui précède la purification des acides nucléiques. Ces étapes de lyse sont bien connues à titre d'exemple indicatif, on peut utiliser les méthodes de lyse telles que décrite dans les demandes de brevet :
- WO-A-00/60049 sur la lyse par sonication,
- WO-A-00/05338 sur la lyse mixte magnétique et mécanique,
- WO-A-99/53304 sur la lyse électrique, et
- WO-A-99/15621 sur la lyse mécanique.
L'homme du métier pourra utiliser d'autres méthodes de lyse bien connues telles que les chocs thermiques ou osmotiques ou les traitements par des agents chaotropiques, tels que les sels de guanidium (brevet US-A-5,234,809).

Cette étape permet généralement de concentrer les acides nucléiques. A titre d'exemple, on peut utiliser des supports solides, tels que des particules magnétiques (voir à ce sujet les brevets US-A-4,672,040 et US-A-5,750,338), et ainsi purifier les acides nucléiques, qui se sont fixés sur ces particules magnétiques, par une étape de lavage. Cette étape de purification des acides nucléiques est particulièrement intéressante si l'on souhaite amplifier ultérieurement lesdits acides nucléiques. Un mode de réalisation particulièrement intéressant de ces particules magnétiques est décrit dans les demandes de brevet WO-A-97/45202 et WO-A-99/35500.

Le terme "support solide" tel qu'utilisé ici inclut tous les matériaux sur lesquels peut être fixé un acide nucléique. Des matériaux de synthèse ou des matériaux naturels, éventuellement modifiés chimiquement, peuvent être utilisés comme support solide, notamment les polysaccharides, tels que les matériaux à base de cellulose, par exemple du papier, des dérivés de cellulose tels que l'acétate de cellulose et la nitrocellulose, ou le dextran ; des polymères, des copolymères, notamment à base de monomères du type styrène, des fibres naturelles telles que le coton, et des fibres synthétiques telles que le nylon ; des matériaux minéraux tels que la silice, le quartz, des verres, des céramiques ; des latex ; des particules magnétiques ; des dérivés métalliques, des gels, etc. Le support solide peut être sous la forme d'une plaque de microtitration, d'une membrane, d'une particule ou d'une plaque sensiblement plane de verre ou silicium ou dérivés.

On peut réaliser l'ensemble du protocole (de l'échantillon prélevé aux amplicons prêts à être hybridé) dans un seul et même tube, traité manuellement ou dans un automate.

Les exemples ci-joints représentent des modes particuliers de réalisation et ne peuvent pas être considérés comme limitant la portée de la présente invention.

Plusieurs contrôles ont été effectués dans ces exemples. Tout d'abord un contrôle négatif avec de l'eau, dans ce cas il n'y a aucun signal ni, pour H5 ni pour N1 qui soit détecté. D'autre part, un contrôle de specificité avec de l'ARN Influenza H3N2, là encore aucun signal pour H5 ou N1 n'a été détecté.

### Exemple 1 : Expérience d'évaluation des amorces H5N1 sur un ARN H5N1 provenant d'échantillon clinique d'Asie :

Les séquences des paires d'oligonucléotides (N1_P1 et N2_P2, utilisées pour l'amplification et la détection de la séquence N1, et H5_P1 et H5_P2, utilisées pour l'amplification et la détection de la séquence H5) et des sondes de détection présentes sous forme de sondes moléculaires (sonde moléculaire N1 et sonde moléculaire H5) sont indiquées ci-dessous :

| | |
|---|---|
| N1_P2 | 5'-GGAATGCTCCTGTTATCCTGA-3' |
| N1_P1 | 5'-**AATTCTAATACGACTCACTATAGGGG**CGTGGATTGTCTCCGAAA-3' |
| N1 Sonde | 5'-CGATCGGCGAAATCACATGTGTGTGCAGGGACGATCG-3' |
| H5_P2 | 5'-GCCGAATGATGCCATCAA-3' |
| H5_P1 | 5'-**AATTCTAATACGACTCACTATAGGGG**TGTATGTTGTGGAATGGCA-3' |
| H5 Sonde | 5'-CGATCGACACCAAGTGTCAAACTCCAATCGATCG-3' |

La séquence indiquée en gras correspond à la séquence promotrice T7, reconnue par l'ARN polymérase T7 et est retrouvée dans les oligonucléotides P1 pour la mise en oeuvre de la technique NASBA.

Le traitement de l'échantillon se fait à l'aide du système miniMAG, comme décrit dans le protocole opératoire. Les kits utilisés sont :
- NucliSens Lysis Buffer, bioMérieux B. V. (Boxtel, Hollande), Lot No. 200295, et
- NucliSens Magnetic Extraction Reagents, bioMérieux B. V., Lot No. #200297.

### Protocole opératoire du test de détection:

Selon les instructions du kit Nuclisens EasyQ Basic Kit (bioMérieux B.V., Boxtel, Hollande, Lot No. #285006), deux mélanges réactionnels, l'un servant à l'amplification et à la détection de la séquence H5 (« mix H5 ») et le second à l'amplification et à là détection de la séquence N1 (« mix N1») ont été préparés. Brièvement, à 64 µl de diluant ont été ajoutés 11 µl d'eau, 13 µl de KCl à 1,2 M, 4 µl de chaque oligonucléotide (H5_P1 et H5_P2 ou N1_P1 et N1_P2, solution mère à 10 µM) et 0,8 µl de la sonde de détection appropriée (la sonde moléculaire H5 ou la sonde moléculaire N1, solution mère à 2 µM). Un volume de 10 µl de chaque mélange a ensuite été ajouté à 5 µl de la cible ARN. Parallèlement, une solution d'enzymes a été préparée, et 5 µl de cette solution ont été ajoutés au mélange réactionnel dans le tube, pour un volume final de 20 µl. Les échantillons ont ensuite été placés dans les conditions de réaction préconisées par le kit Nuclisens EasyQ Basic Kit afin de permettre l'amplification et la détection des séquences d'intérêt par la technique isotherme NASBA (Kievits T. et al. J. Virol. Methods (1991) vol. 35(3) p.273-286).

Afin de valider ce test de détection, les cibles ARN utilisées ont été les suivantes:
- H5N1 : Influenza A sous-type H5N1 Vietnam 1194/2004,
- Influenza A: sous-type H3N2,
- ARN contrôle H5: sous-type H5N3,
- Influenza B.

Les contrôles appropriés (positif et négatif) ont été inclus dans le test.

Après amplification et détection sur le système NucliSens EasyQ, les résultats suivants ont été obtenus :

| **Virus** | **Résultats H5 NASBA** | **Résultats N1 NASBA** |
|---|---|---|
| Contrôle négatif | Négative | Négative |
| Influenza A H3N2 | Négative | Négative |
| Negative | Négative | Négative |
| Flu A H5N1 Vietnam 1194/2004 | **Positive** | **Positive** |
| Contrôle négatif | Négative | Négative |
| Flu A H5N1 Vietnam 1194/2004 | **Positive** | **Positive** |
| Flux A H5N3 Duck control virus | **Positive** | Négative |

Ces résultats montrent que les oligonucléotides et sondes de détection H5N1 sont spécifiques et détectent bien leurs cibles respectives H5 et N1.

### Exemple 2 : Expérience d'évaluation des amorces et sondes de détection pour H5N1 dans un test multiplex :

Dans le cas du multiplex, la sonde moléculaire N1 utilisée est marquée au CY5, alors que la sonde moléculaire H5 reste marquée au FAM.

Pour information afin de démontrer la fonctionnalité de nos séquences dans un test multiplex, on utilise comme cible un transcript synthétique qui a été construit à partir de l'ARN recombinant H5N1. C'est un ARN qui comprend uniquement les régions H5 et N1. Il est utilisé comme ARN référence pour évaluer les performances de nos amorces d'amplification et sondes de détection pour H5 et N1 (comme c'est un transcrit synthétique, on l'a en grande quantité, ce qui nous évite d'utiliser l'ARN recombinant H5N1 qui est très précieux.

Selon les instructions du kit Nuclisens EasyQ Basic Kit (bioMérieux B.V., Boxtel, Hollande, Lot No. #285006), un mélange réactionnel unique permettant la détection simultanée du gène H5 et du gène N1 est préparé. Brièvement, à 64 µl de diluant ont été ajoutés 11 µl d'eau, 13 µl de KCl à 1,2 M, 8 µl d'une solution d'oligonucléotides et de sondes moléculaires (contenant les oligonucléotides H5_P1 et H5_P2 pour H5 à 5 µM, les oligonucléotides N1_P1 et N1_P2 pour N1 à 20 µM, la sonde moléculaire H5-FAM à 2 µM et la sonde moléculaire N1-CY5 à 2 µM). Un volume de 10 µl du mélange a ensuite été ajouté à 5 µl de la cible ARN (transcrit H5N1 à 1000 copies/NASBA).

La différence de concentration entre les amorces H5 et les amorces N1 est sensiblement inversement proportionnelle à la différence de sensibilité. La concentration d' amorces H5 est donc quatre fois inférieure à celle de N1.

Parallèlement, une solution d'enzymes a été préparée, et 5 µl de cette solution ont été ajoutés au mélange réactionnel dans le tube, pour un volume final de 20 µl. Les échantillons ont ensuite été placés dans les conditions de réaction préconisées par le kit Nuclisens EasyQ Basic Kit afin de permettre l'amplification et la détection des séquences d'intérêt par la technique isotherme NASBA (Kievits T. et al. J. Virol. Methods (1991) vol. 35(3) p. 273-286).

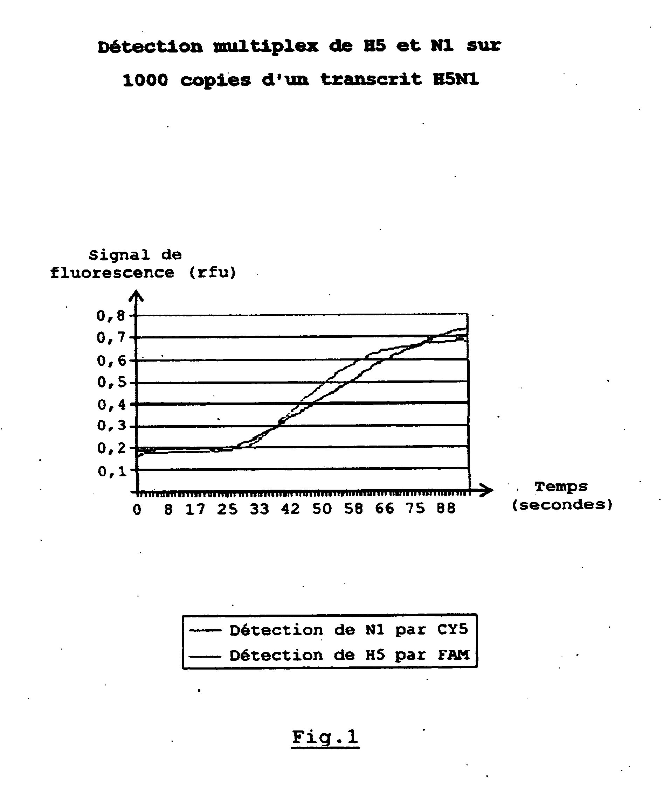

Les résultats sont visibles sur la figure 1. Ces résultats montrent que la détection simultanée de H5 et de N1 en un seul tube fonctionne très bien sur un transcrit synthétique.

## Revendications

1. Double paire d'oligonucléotides destinée à permettre l'amplification de deux séquences cibles localisées respectivement dans le gène H5 et dans le gène N1 du génome du virus *Influenza* A, la paire d'oligonucléotides destinée à l'amplification du gène H5 consistant en :
- un premier oligonucléotide d'une longueur comprise entre 10 et 50 nucléotides et comprenant au moins un fragment de 10 nucléotides consécutifs issus de :
SEQ ID No. 1 : TGTATGTTGTGGAATGGCA, ou sa séquence complémentaire, et
- un second oligonucléotide d'une longueur comprise entre 10 et 50 nucléotides et comprenant au moins un fragment de 10 nucléotides consécutifs issus de :
SEQ ID No. 2 : GCCGAATGATGCCATCAA, ou sa séquence complémentaire,
alors que la paire d'oligonucléotides destinée à l'amplification du gène N1 consiste en :
- un premier oligonucléotide d'une longueur comprise entre 10 et 50 nucléotides et comprenant au moins un fragment de 10 nucléotides consécutifs issus de :
SEQ ID No. 3 : CGTGGATTGTCTCCGAAA, ou sa séquence complémentaire, et
- un second oligonucléotide d'une longueur comprise entre 10 et 50 nucléotides et comprenant au moins un fragment de 10 nucléotides consécutifs issus de :
SEQ ID No. 4 : GGAATGCTCCTGTTATCCTGA, ou sa séquence complémentaire.

2. Paire d'oligonucléotides destinée à permettre l'amplification d'une séquence cible localisée dans le gène H5 du génome du virus *Influenza* A, la paire d'oligonucléotides consistant en :
- un premier oligonucléotide d'une longueur comprise entre 10 et 50 nucléotides et comprenant au moins un fragment de 10 nucléotides consécutifs issus de :
SEQ ID No. 1 : TGTATGTTGTGGAATGGCA, ou sa séquence complémentaire, et
- un second oligonucléotide d'une longueur comprise entre 10 et 50 nucléotides et comprenant au moins un fragment de 10 nucléotides consécutifs issus de :
SEQ ID No. 2 : GCCGAATGATGCCATCAA, ou sa séquence complémentaire.

3. Paire d'oligonucléotides destinée à permettre l'amplification d'une séquence cible localisée dans le gène N1 du génome du virus *Influenza* A, la paire d'oligonucléotides consistant en :
- un premier oligonucléotide d'une longueur comprise entre 10 et 50 nucléotides et comprenant au moins un fragment de 10 nucléotides consécutifs issus de :
SEQ ID No. 3 : CGTGGATTGTCTCCGAAA, ou sa séquence complémentaire,
- un second oligonucléotide d'une longueur comprise entre 10 et 50 nucléotides et comprenant au moins un fragment de 10 nucléotides consécutifs issus de :
SEQ ID No. 4 : GGAATGCTCCTGTTATCCTGA, ou sa séquence complémentaire.

4. Paire(s) d'oligonucléotides, selon l'une quelconque des revendications 1 à 3, **caractérisée(s)** par le fait que le premier oligonucléotide comprend additionnellement une séquence promotrice qui peut être reconnue par une enzyme ARN polymérase ADN dépendante.

5. Paire(s) d'oligonucléotides, selon la revendication 4, **caractérisée(s)** par le fait que la séquence promotrice qui peut être reconnue par une enzyme ARN polymérase ADN dépendante est un promoteur T7.

6. Paire d'oligonucléotides, selon l'une quelconque des revendications 4 ou 5, dans laquelle le premier oligonucléotide, lorsque cet oligonucléotide permet l'amplification de la séquence cible localisée dans le gène H5, est **caractérisé par le fait qu'**il consiste essentiellement en la séquence suivante :
SEQ ID No. 5 : AATTCTAATACGACTCACTATAGGGGTGTATGTTGTGGAATGGCA, ou sa séquence complémentaire.

7. Paire d'oligonucléotides, selon l'une quelconque des revendications 4 à 6, dans laquelle le premier oligonucléotide, lorsque cet oligonucléotide permet l'amplification de la séquence cible localisée dans le gène N1, est **caractérisé par le fait qu'**il consiste essentiellement en la séquence suivante :
SEQ ID No. 6 : AATTCTAATACGACTCACTATAGGGGCGTGGATTGTCTCCGAAA, ou sa séquence complémentaire.

8. Paire d'oligonucléotides, selon l'une quelconque des revendications 1 à 7, **caractérisé par le fait que** chaque oligonucléotide est d'une longueur comprise entre 12 et 30 nucléotides et comprenant au moins un fragment de 16 nucléotides consécutifs, et préférentiellement d'une longueur comprise entre 15 et 26 nucléotides et comprenant au moins un fragment de 18 nucléotides.

9. Paire d'oligonucléotides destinée à être utilisé comme sonde de détection de deux séquences cibles localisées respectivement dans les gènes H5 et N1 du génome du virus *Influenza* A, la sonde destinée à la détection du gène H5 consistant en :
SEQ ID No. 7 : ACACCAAGTGTCAAACTCCAAT, ou sa séquence complémentaire,
alors que la sonde destinée à la détection du gène N1 consiste en :
SEQ ID No. 8 : GCGAAATCACATGTGTGTGCAGGGA, ou sa séquence complémentaire,
chaque séquence comportant au moins un moyen de marquage.

10. Oligonucléotide, destiné à être utilisé comme sonde de détection d'une séquence d'acides nucléiques amplifiée résultant de l'amplification d'une séquence cible localisée dans le gène H5 du génome du virus *Influenza* A, ladite amplification étant réalisée par l'intermédiaire d'une paire d'oligonucléotides selon l'une quelconque des revendications 2, 4 à 8, la sonde de détection étant d'une longueur comprise entre 10 et 50 nucléotides et comprenant au moins un fragment de 10 nucléotides consécutifs issus de :
SEQ ID No. 7 : ACACCAAGTGTCAAACTCCAAT, ou sa séquence complémentaire à l'exception de l'oligonucléotide de séquence CAT TGG AGT TTG ACA CTT GGT GTT, la séquence comportant au moins un moyen de marquage.

11. Oligonucléotide, destiné à être utilisé comme sonde de détection d'une séquence d'acides nucléiques amplifiée résultant de l'amplification d'une séquence cible localisée dans le gène N1 du génome du virus *Influenza* A, ladite amplification étant réalisée par l'intermédiaire d'une paire d'oligonucléotides selon l'une quelconque des revendications 3 à 8, la sonde de détection étant d'une longueur comprise entre 10 et 50 nucléotides et comprenant au moins un fragment de 10 nucléotides consécutifs issus de :
SEQ ID No. 8 : GCGAAATCACATGTGTGTGCAGGGA, ou sa séquence complémentaire, la séquence comportant au moins un moyen de marquage.

12. Sonde de détection, telle que définie dans l'une quelconque des revendications 9 à 11, **caractérisée par le fait qu'**elle est constituée par une sonde moléculaire.

13. Sonde de détection, selon l'une quelconque des revendications 9 à 12, **caractérisée par le fait qu'**elle est constituée par une sonde moléculaire, préférentiellement constitué par :
SEQ ID 9: [6-FAM]-cgatcgaCACCAAGTGTCAAACTCCAAtcgatcg-[DabSyl], pour la détection du gène H5,
SEQ ID 10: [6-FAM]-cgatcgGCGAAATCACATGTGTGTGCAGGGAcgatcg-[DabSyl], pour la détection du gène N1.

14. Utilisation d'une ou deux paires d'oligonucléotides, selon l'une quelconque des revendications 1-8, dans une réaction d'amplification d'acides nucléiques ou comme sonde de détection du génome du virus *Influenza* A suspecté d'être présent dans un échantillon biologique.

15. Méthode de détection d'acides nucléiques du virus d'*Influenza* A susceptible d'être présent dans un échantillon, dans laquelle l'échantillon est soumis à une réaction d'amplification d'acides nucléiques utilisant une paire d'oligonucléotides, selon l'une quelconque des revendications 1 à 8, en présence des réactifs d'amplification nécessaires à une telle amplification et la présence d'amplicons d'intérêt est détecté.

16. Méthode, selon la revendication 15, **caractérisée en ce que** la réaction d'amplification utilisée est une RT-PCR.

17. Méthode, selon la revendication 15, **caractérisée en ce que** la réaction d'amplification utilisée est une technique d'amplification transcriptionnelle.

18. Méthode, selon la revendication 17, **caractérisée en ce que** la réaction d'amplification utilisée est la technique NASBA.

19. Méthode d'amplification de deux gènes H5 et N1 du virus d'*Influenza* A susceptible d'être présent dans un échantillon, comprenant les étapes suivantes :
- incuber l'échantillon dans un tampon d'amplification en présence :
• de deux amorces d'amplification selon la revendication 2, chacune ayant une longueur comprise entre 10 et 50 nucléotides, l'une comprenant additionnellement une séquence promotrice, l'autre de polarité opposée à l'amorce associée à la séquence promotrice, pour s'hybrider respectivement en amont et en aval d'une zone d'intérêt localisée dans le gène H5 du virus d'*Influenza* A,
• de deux amorces d'amplification selon la revendication 3, chacune ayant une longueur comprise entre 10 et 50 nucléotides, l'une comprenant additionnellement une séquence promotrice, l'autre de polarité opposée à l'amorce associée à la séquence promotrice, pour s'hybrider respectivement en amont et en aval d'une zone d'intérêt localisée dans le gène N1 du virus d'*Influenza* A,
- ajouter les réactifs suivants dans l'échantillon:
• une enzyme ayant une activité RNA dépendant DNA polymérase,
• une enzyme ayant une activité DNA dépendant DNA polymérase,
• une enzyme ayant une activité RNase H,
• une enzyme ayant une activité DNA dependant RNA polymérase, et
- maintenir le mélange réactionnel ainsi créé sous des conditions appropriées et pendant une durée de temps suffisante pour qu'une amplification ait lieu.

20. Kit de détection des gènes H5 et N1 du virus *Influenza* A susceptible d'être présent dans un échantillon, contenant :
- deux paires d'oligonucléotides selon les revendications 1 à 8,
- deux oligonucléotides marqués ou pouvant être marqués, selon l'une quelconque des revendications 9 à 13, et ayant une séquence d'acide nucléique substantiellement complémentaire avec au moins une partie de la séquence d'acide nucléique amplifiée,
- des réactifs nécessaires à la réalisation d'une réaction d'amplification.

21. Kit selon la revendication 20, dans lequel les réactifs nécessaires à la réalisation d'une réaction d'amplification sont des réactifs permettant une amplification NASBA.

## Claims

1. Double pair of oligonucleotides for amplifying two target sequences located, respectively, in the H5 gene and in the N1 gene of the genome of the *Influenza* A virus, the pair of oligonucleotides for amplifying the H5 gene consisting of:
- a first oligonucleotide of a length ranging between 10 and 50 nucleotides and comprising at least one fragment of 10 consecutive nucleotides derived from:
SEQ ID No. 1: TGTATGTTGTGGAATGGCA, or the sequence complementary thereto, and
- a second oligonucleotide of a length ranging between 10 and 50 nucleotides and comprising at least one fragment of 10 consecutive nucleotides derived from:
SEQ ID No. 2: GCCGAATGATGCCATCAA, or the sequence complementary thereto,
while the pair of oligonucleotides for amplifying the N1 gene consists of:
- a first oligonucleotide of a length ranging between 10 and 50 nucleotides and comprising at least one fragment of 10 consecutive nucleotides derived from:
SEQ ID No. 3: CGTGGATTGTCTCCGAAA, or the sequence complementary thereto, and
- a second oligonucleotide of a length ranging between 10 and 50 nucleotides and comprising at least one fragment of 10 consecutive nucleotides derived from:
SEQ ID No. 4: GGAATGCTCCTGTTATCCTGA, or the sequence complementary thereto.

2. Pair of oligonucleotides for amplifying a target sequence located in the H5 gene of the genome of the *Influenza* A virus, the pair of oligonucleotides consisting of:
- a first oligonucleotide of a length ranging between 10 and 50 nucleotides and comprising at least one fragment of 10 consecutive nucleotides derived from:
SEQ ID No. 1: TGTATGTTGTGGAATGGCA, or the sequence complementary thereto, and
- a second oligonucleotide of a length ranging between 10 and 50 nucleotides and comprising at least one fragment of 10 consecutive nucleotides derived from:
SEQ ID No. 2: GCCGAATGATGCCATCAA, or the sequence complementary thereto.

3. Pair of oligonucleotides for amplifying a target sequence located in the N1 gene of the genome of the *Influenza* A virus, the pair of oligonucleotides consisting of:
- a first oligonucleotide of a length ranging between 10 and 50 nucleotides and comprising at least one fragment of 10 consecutive nucleotides derived from:
SEQ ID No. 3: CGTGGATTGATTGTCTCCGAAA, or the sequence complementary thereto, and
- a second oligonucleotide of a length ranging between 10 and 50 nucleotides and comprising at least one fragment of 10 consecutive nucleotides derived from:
SEQ ID No. 4: GGAATGCTCCTGTTATCCTGA, or the sequence complementary thereto.

4. Pair(s) of oligonucleotides according to any one of Claims 1 to 3, **characterized in that** the first oligonucleotide additionally comprises a promoter sequence which can be recognized by a DNA-dependent RNA polymerase enzyme.

5. Pair(s) of oligonucleotides according to Claim 4, **characterized in that** the promoter sequence which can be recognized by a DNA-dependent RNA polymerase enzyme is a T7 promoter.

6. Pair of oligonucleotides according to either one of Claims 4 and 5, in which the first oligonucleotide, when this oligonucleotide makes it possible to amplify the target sequence located in the H5 gene, is **characterized in that** it consists essentially of the following sequence:
SEQ ID No. 5:
AATTCTAATACGACTCACTATAGGGGTGTATGTTGTGGAATGGCA, or the sequence complementary thereto.

7. Pair of oligonucleotides according to any one of Claims 4 to 6, in which the first oligonucleotide, when this oligonucleotide makes it possible to amplify the target sequence located in the N1 gene, is **characterized in that** it consists essentially of the following sequence:
SEQ ID No. 6:
AATTCTAATACGACTCACTATAGGGGCGTGGATTGTCTCCGAAA, or the sequence complementary thereto.

8. Pair of oligonucleotides according to any one of Claims 1 to 7, **characterized in that** each oligonucleotide is of a length ranging between 12 and 30 nucleotides and comprising at least one fragment of 16 consecutive nucleotides, and preferably of a length ranging between 15 and 26 nucleotides and comprising at least one fragment of 18 nucleotides.

9. Pair of oligonucleotides for use as a probe for detecting two target sequences located, respectively in the H5 and N1 genes of the genome of the *Influenza* A virus, the probe for detecting the H5 gene consisting of
SEQ ID No. 7: ACACCAAGTGTCAAACTCCAAT, or the sequence complementary thereto,
while the probe for detecting the N1 gene consists of:
SEQ ID No. 8: GCGAAATCACATGTGTGTGCAGGGA, or the sequence complementary thereto,
each sequence comprising at least one labeling means.

10. Oligonucleotide for use as a probe for detecting an amplified nucleic acid sequence resulting from the amplification of a target sequence located in the H5 gene of the genome of the *Influenza* A virus, said amplification being carried out by means of a pair of oligonucleotides according to any one of Claims 2 and 4 to 8, the detection probe being of a length ranging between 10 and 50 nucleotides and comprising at least one fragment of 10 consecutive nucleotides derived from:
SEQ ID No. 7: ACACCAAGTGTCAAACTCCAAT, or the sequence complementary thereto with the exception of the oligonucleotide having the sequence CATTGGAGTTTGACACTTGGTGTT, the sequence comprising at least one labeling means.

11. Oligonucleotide for use as a probe for detecting an amplified nucleic acid sequence resulting from the amplification of a target sequence located in the N1 gene of the genome of the *Influenza* A virus, said amplification being carried out by means of a pair of oligonucleotides according to any one of Claims 3 to 8, the detection probe being of a length ranging between 10 and 50 nucleotides and comprising at least one fragment of 10 consecutive nucleotides derived from:
SEQ ID No. 8: GCGAAATCACATGTGTGTGCAGGGA, or the sequence complementary thereto, the sequence comprising at least one labeling means.

12. Detection probe as defined in any one of Claims 9 to 11, **characterized in that** it is composed of a molecular probe.

13. Detection probe according to any one of Claims 9 to 12, **characterized in that** it is composed of a molecular probe, preferably composed of:
SEQ ID 9:
[6-FAM]-cgatcgaCACCAAGTGTCAAACTCCAAtcgatcg-[DabSyl], for detecting the H5 gene,
SEQ ID 10:
[6-FAM]-cgatcgGCGAAATCACATGTGTGTGCAGGGAcgatcg-[DabSyl], for detecting the N1 gene.

14. Use of one or two pairs of oligonucleotides, according to any one of Claims 1 to 8, in a reaction for the amplification of nucleic acids or as a probe for the detection of the genome of the *Influenza* A virus suspected of being present in a biological sample.

15. Method for detecting nucleic acids of the *Influenza* A virus that may be present in a sample, in which the sample is subjected to a reaction for the amplification of nucleic acids using a pair of oligonucleotides, according to any one of Claims 1 to 8, in the presence of the amplification reagents required for such an amplification, and the presence of amplicons of interest is detected.

16. Method according to Claim 15, **characterized in that** the amplification reaction used is an RT-PCR.

17. Method according to Claim 15, **characterized in that** the amplification reaction used is a transcriptional amplification technique.

18. Method according to Claim 17, **characterized in that** the amplification reaction used is the NASBA technique.

19. Method for amplifying two H5 and N1 genes of the *Influenza* A virus that may be present in a sample, comprising the following steps:
- incubating the sample in an amplification buffer in the presence:
• of two amplification primers according to Claim 2, each having a length ranging between 10 and 50 nucleotides, one additionally comprising a promoter sequence, the other of opposite polarity to the primer associated with the promoter sequence, in order to hybridize respectively upstream and downstream of a region of interest located in the H5 gene of the *Influenza* A virus,
• of two amplification primers according to Claim 3, each having a length ranging between 10 and 50 nucleotides, one additionally comprising a promoter sequence, the other of opposite polarity to the primer associated with the promoter sequence, in order to hybridize respectively upstream and downstream of a region of interest located in the N1 gene of the *Influenza* A virus,
- adding the following reagents to the sample:
• an enzyme having an RNA-dependant DNA polymerase activity,
• an enzyme having a DNA-dependant DNA polymerase activity,
• an enzyme having an RNase H activity,
• an enzyme having a DNA-dependant RNA polymerase activity, and
- maintaining the reaction mix thus created under suitable conditions and for a period of time sufficient for an amplification to take place.

20. Kit for detecting the H5 and N1 genes of the *Influenza* A virus that may be present in a sample, containing:
- two pairs of oligonucleotides according to Claims 1 to 8,
- two oligonucleotides that are labeled or that can be labeled, according to any one of Claims 9 to 13, and that have a nucleic acid sequence substantially complementary with at least one part of the amplified nucleic acid sequence,
- reagents required for carrying out an amplification reaction.

21. Kit according to Claim 20, in which the reagents required for carrying out an amplification reaction are reagents for a NASBA amplification.

## Patentansprüche

1. Oligonukleotiddoppelpaar, das dazu bestimmt ist, die Amplifikation von zwei Zielsequenzen zu ermöglichen, die in dem Gen H5 bzw. dem Gen N1 des Genoms des *Influenza*-A-Virus lokalisiert sind, wobei das Oligonukleotidpaar, das zur Amplifikation des Gens H5 bestimmt ist, aus Folgendem besteht:
- einem ersten Oligonukleotid mit einer Länge im Bereich zwischen 10 und 50 Nukleotiden, das mindestens ein Fragment mit 10 aufeinanderfolgenden Nukleotiden umfasst, abgeleitet aus:
SEQ ID No. 1: TGTATGTTGTGGAATGGCA oder deren komplementärer Sequenz, und
- einem zweiten Oligonukleotid mit einer Länge im Bereich zwischen 10 und 50 Nukleotiden, das mindestens ein Fragment mit 10 aufeinanderfolgenden Nukleotiden umfasst, abgeleitet aus:
SEQ ID No. 2: GCCGAATGATGCCATCAA oder deren komplementärer Sequenz,
während das Oligonukleotidpaar, das zur Amplifikation des Gens N1 bestimmt ist, aus Folgendem besteht:
- einem ersten Oligonukleotid mit einer Länge im Bereich zwischen 10 und 50 Nukleotiden, das mindestens ein Fragment mit 10 aufeinanderfolgenden Nukleotiden umfasst, abgeleitet aus:
SEQ ID No. 3: CGTGGATTGTCTCCGAAA oder deren komplementärer Sequenz, und
- einem zweiten Oligonukleotid mit einer Länge im Bereich zwischen 10 und 50 Nukleotiden, das mindestens ein Fragment mit 10 aufeinanderfolgenden Nukleotiden umfasst, abgeleitet aus:
SEQ ID No. 4: GGAATGCTCCTGTTATCCTGA oder deren komplementärer Sequenz.

2. Oligonukleotidpaar, das dazu bestimmt ist, die Amplifikation einer Zielsequenz zu ermöglichen, die in dem Gen H5 des Genoms des *Influenza*-A-Virus lokalisiert ist, wobei das Oligonukleotidpaar aus Folgendem besteht:
- einem ersten Oligonukleotid mit einer Länge im Bereich zwischen 10 und 50 Nukleotiden, das mindestens ein Fragment mit 10 aufeinanderfolgenden Nukleotiden umfasst, abgeleitet aus:
SEQ ID No. 1: TGTATGTTGTGGAATGGCA oder deren komplementärer Sequenz, und
- einem zweiten Oligonukleotid mit einer Länge im Bereich zwischen 10 und 50 Nukleotiden, das mindestens ein Fragment mit 10 aufeinanderfolgenden Nukleotiden umfasst, abgeleitet aus:
SEQ ID No. 2: GCCGAATGATGCCATCAA oder deren komplementärer Sequenz.

3. Oligonukleotidpaar, das dazu bestimmt ist, die Amplifikation einer Zielsequenz zu ermöglichen, die in dem Gen N1 des Genoms des *Influenza*-A-Virus lokalisiert ist, wobei das Oligonukleotidpaar aus Folgendem besteht:
- einem ersten Oligonukleotid mit einer Länge im Bereich zwischen 10 und 50 Nukleotiden, das mindestens ein Fragment mit 10 aufeinanderfolgenden Nukleotiden umfasst, abgeleitet aus:
SEQ ID No. 3: CGTGGATTGTCTCCGAAA oder deren komplementärer Sequenz,
- einem zweiten Oligonukleotid mit einer Länge im Bereich zwischen 10 und 50 Nukleotiden, das mindestens ein Fragment mit 10 aufeinanderfolgenden Nukleotiden umfasst, abgeleitet aus:
SEQ ID No. 4: GGAATGCTCCTGTTATCCTGA oder deren komplementärer Sequenz.

4. Oligonukleotidpaar(e) nach einem der Ansprüche 1 bis 3, **gekennzeichnet durch** die Tatsache, dass das erste Oligonukleotid zusätzlich eine Promotersequenz umfasst, die **durch** ein DNA-abhängiges RNA-Polymeraseenzym erkannt werden kann.

5. Oligonukleotidpaar(e) nach Anspruch 4, **gekennzeichnet durch** die Tatsache, dass die Promotersequenz, die **durch** ein DNA-abhängiges RNA-Polymeraseenzym erkannt werden kann, eine T7-Promoter ist.

6. Oligonukleotidpaar nach einem der Ansprüche 4 oder 5, wobei das erste Oligonukleotid, wenn dieses Oligonukleotid die Amplifikation der Zielsequenz ermöglicht, die in dem Gen H5 lokalisiert ist, durch die Tatsache **gekennzeichnet** ist, dass es im Wesentlichen aus der folgenden Sequenz besteht:
SEQ ID No. 5:
AATTCTAATACGACTCACTATAGGGGTGTATGTTGTGGAATGGCA oder deren komplementäre Sequenz.

7. Oligonukleotidpaar nach einem der Ansprüche 4 bis 6, wobei das erste Oligonukleotid, wenn dieses Oligonukleotid die Amplifikation der Zielsequenz ermöglicht, die in dem Gen N1 lokalisiert ist, durch die Tatsache **gekennzeichnet** ist, dass es im Wesentlichen aus der folgenden Sequenz besteht:
SEQ ID No. 6:
AATTCTAATACGACTCACTATAGGGGCGTGGATTGTCTCCGAAA oder deren komplementärer Sequenz.

8. Oligonukleotidpaar nach einem der Ansprüche 1 bis 7, **gekennzeichnet durch** die Tatsache, dass jedes Oligonukleotid eine Länge im Bereich zwischen 12 und 30 Nukleotiden hat und mindestens ein Fragment mit 16 aufeinanderfolgenden Nukleotiden umfasst, und vorzugsweise eine Länge im Bereich zwischen 15 und 26 Nukleotiden hat und mindestens ein Fragment mit 18 Nukleotiden umfasst.

9. Oligonukleotidpaar, das dazu bestimmt ist, als Sonde zum Nachweis von zwei Zielsequenzen verwendet zu werden, die in den Genen H5 bzw. N1 des Genoms des *Influenza*-A-Virus lokalisiert sind, wobei die Sonde, die zum Nachweis des Gens H5 bestimmt ist, aus Folgendem besteht:
SEQ ID No. 7: ACACCAAGTGTCAAACTCCAAT oder deren komplementärer Sequenz,
während die Sonde, die zum Nachweis des Gens N1 bestimmt ist, aus Folgendem besteht:
SEQ ID No. 8: GCGAAATCACATGTGTGTGCAGGGA oder deren komplementärer Sequenz,
wobei jede Sequenz mindestens ein Mittel zur Markierung umfasst.

10. Oligonukleotid, das dazu bestimmt ist, als Sonde zum Nachweis einer amplifizierten Nukleinsäuresequenz verwendet zu werden, die aus der Amplifikation einer Zielsequenz resultiert, die in dem Gen H5 des Genoms des *Influenza*-A-Virus lokalisiert ist, wobei die Amplifikation mittels eines Oligonukleotidpaars nach einem der Ansprüche 2, 4 bis 8 ausgeführt wird, wobei die Sonde zum Nachweis eine Länge im Bereich zwischen 10 und 50 Nukleotiden hat und mindestens ein Fragment mit 10 aufeinanderfolgenden Nukleotiden umfasst, abgeleitet aus:
SEQ ID No. 7: ACACCAAGTGTCAAACTCCAAT oder deren komplementärer Sequenz, mit Ausnahme des Oligonukleotids mit der Sequenz CATTGGAGTTTGACACTTGGTGTT, wobei die Sequenz mindestens ein Mittel zur Markierung beinhaltet.

11. Oligonukleotid, das dazu bestimmt ist, als Sonde zum Nachweis einer amplifizierten Nukleinsäuresequenz verwendet zu werden, die aus der Amplifikation einer Zielsequenz resultiert, die in dem Gen N1 des Genoms des *Influenza*-A-Virus lokalisiert ist, wobei die Amplifikation mittels eines Oligonukleotidpaars nach einem der Ansprüche 3 bis 8 ausgeführt wird, wobei die Sonde zum Nachweis eine Länge im Bereich zwischen 10 und 50 Nukleotiden hat und mindestens ein Fragment mit 10 aufeinanderfolgenden Nukleotiden umfasst, abgeleitet aus:
SEQ ID No. 8: GCGAAATCACATGTGTGTGCAGGGA oder deren komplementärer Sequenz, wobei die Sequenz mindestens ein Mittel zum Markieren beinhaltet.

12. Sonde zum Nachweis, wie in einem der Ansprüche 9 bis 11 definiert, **gekennzeichnet durch** die Tatsache, dass sie aus einer Molekularsonde besteht.

13. Sonde zum Nachweis nach einem der Ansprüche 9 bis 12, **gekennzeichnet durch** die Tatsache, dass sie aus einer Molekularsonde besteht, die vorzugsweise aus:
SEQ ID 9: [6-FAM]-cgatcgaCACCAAGTGTCAAACTCCAAtcgatcg-[DabSyl], zum Nachweis des Gens H5,
SEQ ID 10:
[6-FAM]-cgatcgGCGAAATCACATGTGTGTGCAGGGAcgatcg-[DabSyl], zum Nachweis des Gens N1 besteht.

14. Verwendung von einem oder zwei Oligonukleotidpaaren nach einem der Ansprüche 1 bis 8 in einer Reaktion zur Amplifikation von Nukleinsäuren oder als Sonde zum Nachweis des Genoms des *Influenza*-A-Virus, das in einer biologischen Probe vorhanden sein kann.

15. Verfahren zum Nachweis von Nukleinsäuren des *Influenza*-A-Virus, das in einer Probe vorhanden sein kann, wobei die Probe einer Reaktion zur Amplifikation von Nukleinsäuren unter Verwendung eines Oligonukleotidpaars nach einem der Ansprüche 1 bis 8 in Gegenwart von Amplifikationsreagentien unterzogen wird, die für eine solche Amplifikation erforderlich sind, und die Gegenwart von interessierenden Amplikons nachgewiesen wird.

16. Verfahren nach Anspruch 15, **dadurch gekennzeichnet, dass** die verwendete Reaktion zur Amplifikation eine RT-PCR ist.

17. Verfahren nach Anspruch 15, **dadurch gekennzeichnet, dass** die verwendete Reaktion zur Amplifikation eine transkriptionelle Amplifikationstechnik ist.

18. Verfahren nach Anspruch 17, **dadurch gekennzeichnet, dass** die verwendete Reaktion zur Amplifikation die NASBA-Technik ist.

19. Verfahren zur Amplifikation von zwei Genen, H5 und N1, des *Influenza*-A-Virus, das in einer Probe vorhanden sein kann, das die folgenden Schritte umfasst:
- Inkubieren der Probe in einem Amplifikationspuffer in Gegenwart:
• von zwei Amplifikationsprimern nach Anspruch 2, wobei jeder eine Länge im Bereich zwischen 10 und 50 Nukleotiden hat, wobei der eine zusätzlich eine Promotersequenz umfasst, der andere mit gegensinniger Polarität zum Primer, der mit der Promotersequenz assoziiert ist, um strangaufwärts bzw. strangabwärts einer interessierenden Zone zu hybridisieren, die in dem Gen H5 des *Influenza*-A-Virus lokalisiert ist,
• von zwei Amplifikationsprimern nach Anspruch 3, wobei jeder eine Länge im Bereich zwischen 10 und 50 Nukleotiden hat, wobei der eine zusätzlich eine Promotersequenz umfasst, der andere mit gegensinniger Polarität zum Primer, der mit der Promotersequenz assoziiert ist, um strangaufwärts bzw. strangabwärts einer interessierenden Zone zu hybridisieren, die in dem Gen N1 des *Influenza*-A-Virus lokalisiert ist,
- Zugeben der folgenden Reagentien zur Probe:
• ein Enzym, das eine RNA-abhängige DNA-Polymerase-Aktivität hat,
• ein Enzym, das eine DNA-abhängige DNA-Polymerase-Aktivität hat,
• ein Enzym, das eine RNase-H-Aktivität hat,
• ein Enzym, das eine DNA-abhängige RNA-Polymerase-Aktivität hat, und
- Erhalten des so erzeugten Reaktionsgemischs unter geeigneten Bedingungen und für einen Zeitraum, der ausreicht, damit eine Amplifikation stattfindet.

20. Kit zum Nachweis der Gene H5 und N1 des *Influenza*-A-Virus, das in einer Probe vorhanden sein kann, enthaltend:
- zwei Oligonukleotidpaare nach den Ansprüchen 1 bis 8,
- zwei Oligonukleotide, die markiert sind oder markiert werden können, nach einem der Ansprüche 9 bis 13, und die eine Nukleinsäuresequenz haben, die im Wesentlichen zu mindestens einem Teil der amplifizierten Nukleinsäuresequenz komplementär ist,
- Reagentien, die für die Ausführung einer Amplifikationsreaktion erforderlich sind.

21. Kit nach Anspruch 20, wobei die Reagentien, die für die Ausführung einer Amplifikationsreaktion erforderlich sind, Reagentien sind, die eine NASBA-Amplifikation ermöglichen.
